# EUROPEAN PATENT APPLICATION

(11) **EP 2 187 215 A1**
(43) Date of publication of application: **19.05.2010**
(21) Application number: 08450181.6
(22) Date of filing: 12.11.2008
(51) Int. Cl.: G01N 33/569

(54) **Histomonas meleagridis assay**

(71) Applicant: Veterinärmedizinische Universität Wien, 1210 Vienna (AT)
(72) Inventor: Hess, Michael, 1020 Vienna (AT)
(74) Representative: Sonn & Partner Patentanwälte

(57) **Abstract**

The present invention relates to a method for the detection or quantification of antibodies against *H. meleagridis* comprising
• providing a surface (a) with immobilized antibodies against *H. meleagridis* and *H. meleagridis* antigens immobilized on said antibodies, and/or (b) with isolated *H. meleagridis* antigens,
• contacting a sample which potentially comprises antibodies against *H. meleagridis,* and
• detecting binding events of the antibodies of the sample onto the surface,
uses of this method to diagnose a *H*. *meleagridis* infection, kits and means for said method.

## Description

The present invention relates to the field of pathogen diagnostics in vertebrates.

Histomonosis (syn. histomoniasis, blackhead disease) is an disease of particular significance in poultry caused by the parasitic flagellated protozoon *Histomonas meleagridis.* For a long time the diagnosis of the disease was mainly based on clinical signs and the typical pathomorphological lesions in the caeca and liver. Direct microscopic investigation of caecal content or excreted faeces and certain tissue staining techniques proved to be very time consuming. Furthermore, specific detection could be hampered due to the presence of unspecific contaminants or other flagellates, such as Tetratrichomonas gallinarium or Blastocystis sp..

The ban of chemotherapeutics in animal fodder followed by the re-emergence of the disease has started the search for new and improved diagnostic methods. Most of the currently available diagnostic tools are aiming for detection of the parasite or its DNA, to obtain more information about the pathogenesis of the disease. In this context in-situ hybridization and immunohistology were developed to localize parasitic DNA or parasitic cells in tissue samples. Moreover, the first polymerase chain reaction (PCR) assays for the diagnosis of histomonosis were developed recently, offering the possibility to detect parasitic DNA in all kind of materials including organ samples and faeces. However, using reisolation intermittent excretion of histomonads in faecal samples was demonstrated, which would limit PCR for epidemiological investigations since the pathogen itself is only periodically detectable.

The EP 1 721 965 A1 discloses the first clonal cultures of *H. meleagridis,* Tetratrichomonas gallinarium and Blastocystis sp.. Prior to the development of clonal cultures proper identification of these pathogens was hampered by similar morphological stages shared by these parasites.

For *H.* meleagridis a system capable of distinguishing between infected and non-infected live animals based on a serological read out with a sufficient sensitivity is still not available. It is therefore a goal of the present invention to provide a method capable to reliably detect *H. meleagridis.*

The present invention provides therefore in a first aspect a method for the detection or quantification of antibodies against *Histomonas meleagridis* in a sample comprising
- providing a carrier surface (a) with immobilized antibodies against *Histomonas meleagridis* and *Histomonas meleagridis* antigens immobilized on said antibodies, and/or (b) with isolated *Histomonas meleagridis* antigens, preferably surface antigens,
- contacting a sample which potentially comprises antibodies against *Histomonas meleagridis,* and
- detecting binding events of the antibodies of the sample onto the carrier surface.
According to the invention in order to set up a *H. meleagridis* immunoassay system, microtiter plates were coated with purified histomonads as antigen fraction. However, it turned out that supposed negative sera processed with this type of immunoassay showed a very high background signal. This problem was significantly minimized using the immobilized antibody against *Histomonas meleagridis* as coating antibody in the assay or by using isolated antigens. Furthermore, it was possible to increase the precision of the OD values using this system, displayed in a far lower standard deviation within the tested negative controls.

Without being limited to a specific theory it is believed that the limited results with the assay where *H. meleagridis* is directly coated onto the surface instead of the inventive coating via antibody or use of specific isolated *H. meleagridis* surface antigens are related to the fact that *H. meleagridis* is propagated as a xenic protozoan. Isolates of *H. meleagridis* are usually contaminated by further bacteria. Immobilization of such a heterogenic material onto a surface will usually result in binding reactions of different antibodies specific for a multitude of epitopes, not necessary being specific for *H. meleagridis*. With the inventive set up specificity could be significantly increased and background signals were minimized to tolerable levels. The background of prior attempts reached values where a meaningful diagnosis would be hampered or rendered impossible. Usually birds as the prime target for the assay of the present invention have high antibody titers against xenic bacteria, such as E. Coli or Clostridia. Therefore an assay in birds would be significantly biased to a level where a meaningful interpretation of the results would be impossible. Thus, the present invention provides for the first time an immunoassay suitable to distinctly detect *H. meleagridis* and diagnose *H. meleagridis* infections.

A further benefit of the present invention is the detection of *H. meleagridis* antibodies in the sera of infected hosts. In particular poultry antibody titers can be continuously detected at least after 14 days after an infection or even earlier. Contrary thereto direct detection methods of the parasite e.g. via PCR or direct microscopic investigation of caecal content or excreted faeces can only detect *H. meleagridis* in an acute surge of the infection but not in intermittent periods where the parasite remains quiescent. This drawback of direct methods does not affect an indirect immunoassay method since antibody titers can be continuously detected. Furthermore, it has been shown in the examples that *H. meleagridis* rendered non-pathogenic e.g. after passaging (such as after 100 passages) can be distinguished from an infection of pathogenic *H. meleagridis* (such as with no or few, e.g. lower than 40, lower than 30 or lower than 20 passages).

In a preferred embodiment of the present invention the *Histomonas meleagridis* antigens immobilized via the antibody (a) are *Histomonas meleagridis* cells or cell lysate antigens. The cells immobilized onto the carrier surface via the antibody is sufficiently specific to bind anti-*H. meleagridis* antibodies of a sample. Thus, a specific immunoassay is possible by using complete cells which comprise a multitude of different surface antigens of *H. meleagridis.* In further embodiments it is possible to also use cell lysate antigens which are bound to the immobilized antibodies (a). The antibodies (a) specifically bind surface antigens of *H. meleagridis* from the cell lysate. This cell lysate could furthermore be fractioned before immobilization onto the carrier surface by selecting e.g. the membrane fraction including surface membrane proteins, in particular surface antigens.

It has been found that polyclonal antibodies produced in e.g. rabbits directed against *H. meleagridis* are specific for surface antigens and can be used to bind *H. meleagridis* cells or cell lysate antigens onto the carrier surface. In general, these antibodies (a) can e.g. be polyclonal or monoclonal, directed against complete *H. meleagridis* cells or directed against *H. meleagridis* surface antigens. In the case of monoclonal antibodies it is also possible to use multiple monoclonal antibodies, e.g. directed against 2, 3, 5, 6, 7, 8 or more antigens or epitopes of *H. meleagridis.* The polyclonal, but also a monoclonal, antibody can be specific for, i.e. directed against, *H. meleagridis* cells in general or specific for individual antigens, in particular surface antigens, of *H. meleagridis.* The antibodies (a) are then immobilized onto the carrier surface e.g. via absorption or covalent linkage. For this immobilization it is possible to use commonly used immobilization reagents or linkers such as avidin-biotin, commonly used in the field.

One main benefit of the present invention is that by using the antibody (a) *H. meleagridis* can be specifically immobilized onto the carrier surface to be used in the method of the present invention. The specific immobilization of the H. meleagridis cells, or *H. meleagridis* antigens via antibody linkage has the benefit that xenic bacteria usually found in H. meleagridis preparation and isolates are not bound onto the carrier. Therefore, in a preferred embodiment of the present invention the *Histomonas meleagridis* antigens immobilized on the carrier surface via the antibody (a) are bacteria-free or mask optionally present bacteria. Preferably, bacteria, such as bacteria of xenic cultures, or generally pathogens apart from *H. meleagridis* are below the detection limit using an immunoassay with e.g. fluorescence labeled antibodies or enzyme linked labels as detection means. "Masking" means that a certain level of other bacteria can be tolerated which, however, do not adversely affect the detection method of *H. meleagridis* of the present invention i.e. the detection limit, the standard deviation or the detected background are not increased. Preferably such additional bacteria contaminants are below 10%, below 5%, below 2%, below 1% of the immobilized *H. meleagridis* amount.

In order to prevent cross-reactivities in the detection phase it is preferred that the immobilized antibodies (a) are of a different organism than the sample, preferably the immobilized antibodies (a) are of a mammal, preferably a rabbit. In cases where e.g. the sample is of avian origin it is thus preferred if the immobilized antibodies (a) are e.g. of a mammal. If e.g. the sample is of a bird and anti-avian antibodies are used as detection means, e.g. labeled with a fluorescence label or enzyme label, no cross-reactivities with immobilized antibodies (a) used for the immobilization of the *H. meleagridis* antigens can occur. This differentiation of the origin of the antibodies of the sample and of the carrier surface is preferably on the class-level (birds, mammals) but can also occur on the species level.

In an alternative to immobilizing H. meleagridis antigens via an antibody it is also possible to use isolated surface antigens directly onto the carrier surface if no further xenic bacteria are bound. Therefore, complete H. meleagridis cells or cell lysates are, according to the present invention, not bound to the carrier surface but only isolated surface antigens thereof without or with low bacteria contamination, as mentioned above below the masking level. These isolated surface antigens are preferably purified from contaminating xenic bacteria, in particular preferred is the use of antigens of *H. meleagridis* of recombinant origin. Therefore, in another embodiment of the present invention the isolated surface antigens (b) are purified or recombinant surface antigens. Recombinant surface antigens can be produced in common expression systems using expression vectors comprising one or more genes of *H. meleagridis* surface antigens.

The binding event of the antibodies of the samples onto the carrier surface, in particular the immobilized *H. meleagridis* antigens, such as *H. meleagridis* cells, cell lysate antigens and surface antigens, can be performed by any means known in the art. In a preferred embodiment the binding event is detected by competitive binding of a labeled antibody, preferably fluorescence labeled or enzyme labeled antibody, being specific for immobilized antigens (a) or (b), to the antigens (a) or (b) of the carrier surface during or after the contacting step with the sample. According to this detection method a labeled competitive antibody competes with the antibodies of the samples for the binding sites on the carrier surface. These competitive antibodies with a certain concentration can be mixed with the sample before application onto the solid surface or during the contacting step of the sample with antigens (a) and (b) of the solid surface. Low antibody concentrations in a sample will lead to a higher signal due to increased binding of the labeled antibody onto the solid surface whereas a high antibody concentration in the sample will lead to decreased binding of the competitive antibodies and thus a lower signal. These competitive antibodies can be of the same organism to be analysed or of a different organism. Preferably the competitive antibodies are of a mammal, in particular of a rodent, or of a bird.

In addition or as an alternative the binding event can be detected by secondary antibodies which are specific for the Fc portion of the antibodies to be detected in the sample, and said secondary antibodies are labeled, preferably fluorescence labeled or enzyme labeled. It is thus possible to detect the antibodies of the sample by using secondary antibodies which bind to these antibodies of the sample and then generate a signal. Antibodies of a biological sample contain constant portions (Fc) which can be uniformly targeted by secondary antibodies. The signal is then directly proportional to the antibodies of the sample. These secondary antibodies are preferably mammal antibodies, in particular antibodies of a rodent such as mice, rats, hamsters or rabbits, or of a bird, in particular chicken or turkey, preferably obtained from eggs.

The label of the competitive or secondary antibodies can be any label known in the art, in particular fluorescence labels or enzyme labels. If enzyme labels are used, these enzymes activity preferably generates a detectable signal. Such an enzyme is e.g. horseradish peroxidase.

The sample to be detected can be a blood, blood plasma or blood serum sample. In such a sample abundance of the antibody against *H. meleagridis* can be expected at sufficient concentrations in infected organisms. After an infection the organism usually develops antibodies against *H. meleagridis* found in blood, blood plasma or blood serum in a continuous manner and does not suffer from intermittent acute phases of an infection. Preferably the organism, or a patient, is a mammal or a bird, in particular a chicken, turkey, peafowl, quail, duck or pheasant.

In further preferred embodiments the *Histomonas meleagridis* antigens immobilized on said antibodies (a) or isolated *Histomonas meleagridis* surface antigens (b) are of a clonal culture of *Histomonas meleagridis.* Preferably the culture is of Hm/Turkey/Austria/2922-C6/04, Hm/Turkey/Austria/5642-C4/05, Hm/Turkey/Austria/2877-C3/05, Hm/Turkey/Austria/2877-C4/05, Hm/Turkey/Austria/2877-C7/05, Hm/Turkey/Austria/2877-C11/05,
Hm/Turkey/Austria/2877-C17/05, Hm/Turkey/Germany/4114-C3/05, Hm/Turkey/Germany/4114-C16/05, Hm/Turkey/Germany/4114-C18/05, Hm/Chicken/Hungary/5009-C2/05, Hm/Chicken/Hungary/5009-C7/05, Hm/Chicken/Hungary/5009-C10/05, Hm/Chicken/Hungary/5009-C13/05, Hm/Chicken/Hungary/5009-C14/05, Hm/Chicken/Austria/8175-C2/06, Hm/Chicken/Austria/8175-C3/06, Hm/Chicken/Austria/8175-C5/06, Hm/Chicken/Austria/8175-C7/06, Hm/Chicken/Austria/8175-C18/06, Hm/Chicken/Austria/8175-C20/06, Hm/Turkey/Austria/2877-CH3/05, Hm/Turkey/Austria/2877-C10/05. The isolation of clonal culture of *H. meleagridis* is e.g. disclosed in the EP 1 721 965 A1. It has been found that prior *H. meleagridis* cultures could be contaminated by other flagellates due to similar appearance and behaviour. By using a clonal culture of *H. meleagridis* is is guaranteed that only *H. meleagridis* antigens are immobilized onto the carrier surface. The *H. meleagridis* culture is preferably of an infectious *H. meleagridis.* In other embodiments it is also possible to use attenuated *H. meleagridis,* in particular if the surface antigens are not modified by the attenuation. Such attenuation usually occurs e.g. after passaging.

In a further aspect the present invention provides a method for the diagnosis of an *Histomonas meleagridis* infection in an animal comprising
- providing a blood sample of the animal,
- detecting or quantifying antibodies against *Histomonas meleagridis* in said sample according to a method of any previous claim, and
- comparing amounts of the detected of quantified antibody with antibody amounts of a confirmed infectious state or confirmed non-infectious state amount, thereby diagnosing the *Histomonas meleagridis* infection in the animal.

The inventive method for the detection or quantitation of antibodies against *H. meleagridis* in a sample can be used to diagnose a *H. meleagridis* infection in an organism, animal or human. Animal according to the present invention should be considered as a non-human animal, however the inventive method is of course also suitable for human samples. Preferably the animal is a mammal, such as a rodent, in particular a mouse, rat, hamster or rabbit, or a bird, preferably a bird, in particular preferred chicken, turkey, pigeon, peafowl, quail or pheasant. In particular in preferred embodiments the animal is a chicken wherein a *H. meleagridis* infection can remain dormant or unnoticed if not detected by suitable methods such as of the present invention. In turkeys a *H. meleagridis* infection is most deadly. Thus, in turkeys the present method provides an earlier identification of a *H. meleagridis* infection.

In order to diagnose a *H. meleagridis* infection the detected or quantified antibodies of the samples are preferably compared with amounts of a confirmed infectious state or a confirmed non-infectious state. Preferably the amounts in the sample and in the infectious and non-infectious states are corrected by the standard deviation or two times the standard deviation or three times the standard deviation. If the amount in the sample is above the amount of a non-infectious sample, in particular above 29%, above 58%, above 89% or above 100% of the *H. meleagridis* antibody level of the non-infectious state, a significant infection can be diagnosed in the sample and thus in the animal or human from which the sample derives. In addition or alternatively the amount in the sample can be compared with a confirmed infectious state. If the amount in the sample is within e.g. 70%, within 50%, or within 25% of the amount measured in a confirmed infectious state the sample can be diagnosed as being infected.

In a further aspect the present invention provides a carrier surface with (a) immobilized antibodies against *Histomonas meleagridis* and *Histomonas meleagridis* antigens immobilized on said antibodies, and/or (b) isolated *Histomonas meleagridis* surface antigens.

This carrier surface is suitable for the above mentioned method and can be used for the inventive detection or diagnostic assays. Such a carrier surface is preferably a solid surface or a semi-solid surface, preferably a gel. The carrier surface is preferably inert and/or passivated to prevent unspecific binding of the sample contents.

In further embodiments the carrier surface can e.g. provide distinctive areas with the immobilized *H. meleagridis* antigens (a) or (b). Such areas as preferably in wells or microwells suitable for uptake of solutions e.g. sample solutions, buffer solutions and secondary or competitive antibody solutions.

In further embodiments this carrier surface is defined as mentioned above, in particular *Histomonas meleagridis* antigens immobilized via the antibody (a) may be *Histomonas meleagridis* cells or cell lysate antigens. Preferably the *Histomonas meleag*ridis antigens immobilized on the surface via the antibody (a) are bacteria-free or mask optionally present bacteria. In particular preferred the isolated surface antigens (b) are purified or recombinant antigens. The invention also provides a method of producing the carrier surface comprising immobilizing (a) antibodies against *H. meleagridis* onto a carrier surface and *H. meleagridis* antigens on said antibodies or (b) isolated *H. meleagridis* surface antigens onto the carrier surface.

According to a further aspect the present invention provides a kit suitable for a method described herein comprising a carrier surface mentioned above and detection means for the detection of binding events of antibodies onto the immobilized antigens (a) or (b) of the carrier surface.

Preferably the kit provides all the means to perform the method of the present invention which are not usually found in an average laboratory. For ease of use the kit may also provide means for transportation of the means for the inventive method in order to perform the methods on the spot. Thus the kit may also provide the necessary reagents, in particular solutions of the secondary or competitive antibodies for the detection, sample holding means, washing buffers and/or inoculation buffers. In the preferred embodiment of the kit the detection means are labeled competitive antibodies, preferably fluorescence labeled or enzyme labeled antibodies, being specific for immobilized antigens (a) or (b), labeled secondary antibodies, preferably fluorescence labelled or enzyme labeled antibodies, being specific for the Fc potion of antibodies of an organism expected to be in a sample for which the kit is intended, preferably secondary labeled anti-chicken or anti-turkey antibodies. The kit may further comprise sample holding means, preferably sample isolation and holding means, in particular a syringe, suitable to obtain a sample from an animal or human.

The present invention also provides the use of the carrier surface according to the present invention or the kit described above for a method of a detection or quantifying antibodies against *H. meleagridis* in a sample or diagnosing a *H. meleagridis* infection in an animal or in human.

In preferred embodiments the present invention is defined as follows:
**Definition 1:** A method for the detection or quantification of antibodies against *Histomonas meleagridis* in a sample comprising
   - providing a carrier surface (a) with immobilized antibodies against *Histomonas meleagridis* and *Histomonas meleagridis* antigens immobilized on said antibodies, and/or (b) with isolated *Histomonas meleagridis* antigens,
   - contacting a sample which potentially comprises antibodies against *Histomonas meleagridis,* and
   - detecting binding events of the antibodies of the sample onto the carrier surface.
**Definition 2:** Method according to definition 1, **characterized in that** the *Histomonas meleagridis* antigens immobilized via the antibody (a) are *Histomonas meleagridis* cells or cell lysate antigens.
**Definition 3:** Method according to definitions 1 or 2, **characterized in that** *Histomonas meleagridis* antigens immobilized on the carrier surface via the antibody (a) are bacteria-free or mask optionally present bacteria.
**Definition 4:** Method according to any one of definitions 1 to 3, **characterized in that** the immobilized antibodies (a) are of a different organism than the sample, preferably the immobilized antibodies (a) are of a mammal, preferably a rabbit.
**Definition 5:** Method according to definition 1, **characterized in that** the isolated surface antigens (b) are purified or recombinant antigens.
**Definition 6:** Method according to any one of definitions 1 to 5, **characterized in that** the binding event is detected by competitive binding of a labeled antibody, preferably fluorescence labeled or enzyme labeled antibody, being specific for immobilized antigens (a) or (b), to the antigens (a) or (b) of the carrier surface during or after the contacting step with the sample.
**Definition 7:** Method according to any one of definitions 1 to 6, **characterized in that** the binding event is detected by secondary antibodies which are specific for the Fc portion of the antibodies to be detected in the sample, and said secondary antibodies are labeled, preferably fluorescence labeled or enzyme labeled.
**Definition 8:** Method according to any one of definitions 1 to 7, **characterized in that** the sample is a blood, blood plasma or blood serum sample.
**Definition 9:** Method according to any one of definitions 1 to 8, **characterized in that** the *Histomonas meleagridis* antigens immobilized on said antibodies (a) or isolated *Histomonas melea*gridis surface antigens (b) are of a clonal culture of *Histomonas meleagridis*, preferably
**Definition 10:** Method according to claim 9, **characterized in that** the culture of *Histomonas meleagridis* is of Hm/Turkey/Austria/2922-C6/04, Hm/Turkey/Austria/5642-C4/05, Hm/Turkey/Austria/2877-C3/05, Hm/Turkey/Austria/2877-C4/05, Hm/Turkey/Austria/2877-C7/05, Hm/Turkey/Austria/2877-C11/05, Hm/ Turkey/Austria/2877-C17/05, Hm/Turkey/Germany/4114-C3/05, Hm/Turkey/Germany/4114-C16/05, Hm/Turkey/Germany/4114-C18/05, Hm/Chicken/Hungary/5009-C2/05, Hm/Chicken/Hungary/5009-C7/05, Hm/Chicken/Hungary/5009-C10/05, Hm/Chicken/Hungary/5009-C13/05, Hm/Chicken/Hungary/5009-C14/05, Hm/Chicken/Austria/8175-C2/06, Hm/Chicken/Austria/8175-C3/06, Hm/Chicken/Austria/8175-C5/06, Hm/Chicken/Austria/8175-C7/06, Hm/Chicken/Austria/8175-C18/06, Hm/Chicken/Austria/8175-C20/06, Hm/Turkey/Austria/2877-CH3/05, Hm/Turkey/Austria/2877-C10/05.
**Definition 11:** A method for the diagnosis of an *Histomonas meleagridis* infection in an animal comprising
   - providing a blood sample of the animal,
   - detecting or quantifying antibodies against *Histomonas meleagridis* in said sample according to a method of any previous claim, and
   - comparing amounts of the detected of quantified antibody with antibody amounts of a confirmed infectious state or confirmed non-infectious state amount, thereby diagnosing the *Histomonas meleagridis* infection in the animal.
**Definition 12:** Method according to definition 11, **characterized in that** the animal is a mammal or a bird, preferably a bird, in particular preferred chicken, turkey, peafowl, quail, duck or pheasant.
**Definition 13:** A carrier surface with (a) immobilized antibodies against *Histomonas meleagridis* and *Histomonas meleagridis* antigens immobilized on said antibodies, and/or (b) isolated *Histomonas meleagridis* surface antigens.
**Definition 14:** Carrier surface according to definition 13 being a solid surface or a semi-solid surface, preferably a gel.
**Definition 15:** Carrier surface according to definition 13 or 14, **characterized in that** the *Histomonas meleagridis* antigens immobilized via the antibody (a) are *Histomonas meleagridis* cells or cell lysate antigens.
**Definition 16:** Carrier surface according to any one of definitions 13 to 15, **characterized in that** *Histomonas meleagridis* antigens immobilized on the surface via the antibody (a) are bacteria-free or mask optionally present bacteria.
**Definition 17:** Carrier surface according to definition 13 or 14, **characterized in that** the isolated surface antigens (b) are purified or recombinant antigens.
**Definition 18:** A kit suitable for a method according to definition 1 comprising a carrier surface according to any one of definitions 13 to 17 and detection means for the detection of binding events of antibodies onto the immobilized antigens (a) or (b) of the carrier surface.
**Definition 19:** Kit according to definition 18 wherein said detection means are labeled competitive antibodies, preferably fluorescence labeled or enzyme labeled antibodies, being specific for immobilized antigens (a) or (b) or labeled secondary antibodies, preferably fluorescence labeled or enzyme labeled antibodies, being specific for the Fc potion of antibodies of an organism expected to be in a sample for which the kit is intended, preferably secondary labeled anti-chicken or anti-turkey antibodies.
**Definition 20:** Kit according to definitions 18 or 19 further comprising sample holding means, preferably sample isolation and holding means, in particular a syringe.
**Definition 21:** Use of a carrier surface according to any one of definitions 13 to 17 or a kit according to any one of definitions 18 to 20 in a method according to any one of definitions 1 to 12.

The present invention is further illustrated by the following figures and examples.

### Figures:

Fig. 1. Comparison of the indirect ELISA and the indirect sandwich ELISA measuring sera of non-infected chickens (n=37) and turkeys (n=62). Circles represent outliers.
Fig. 2. OD values of sera obtained from 14 day-old chickens which were infected either directly (n=10) or kept as in-contact (n=4) birds. The sera were tested weekly over 6 weeks beginning with the day of the infection (day 0). Circles represent outliers.
Fig. 3. IgG antibody levels determined in turkey sera collected from birds vaccinated at 14 days of life with histomonads resembling different levels of attenuation (P95, P215 and P295) obtained by continuous passaging. The birds were challenged 4 weeks later with virulent *Histomonas meleagridis.* Testing started at day of the vaccination (day 0) and was performed until 4 weeks post challenge. *= day of challenge with virulent parasites. Circles represent outliers.
Fig. 4. OD values measured in turkey sera of non-vaccinated turkeys infected as challenge control at 6 weeks of age. Data are shown until day 21 p.i., the final day for blood sampling. Day 0 is the day of infection.

### Examples:

### Example 1: Materials and Methods

### Rabbit serum

The immunizing antigen for the rabbits was the clonal culture *H. meleagridis* (Hm/Turkey/Austria/2922-66/C04), originally isolated from a diseased turkey and established through microma-nipulation (Hess et al., 2006b). The development of the polyclonal histomonad antiserum in rabbits used for coating the microtiter plates was described in detail previously (Singh et al., 2008). The culture was also used in the animal experiments as described below. Altogether the rabbits were injected four times with six weeks intervals. Six weeks after the last injection the animals were euthanized, blood was collected, centrifuged and the serum was stored at -20° for further use.

### Chicken and turkey sera from non-infected birds

A total of 37 sera from spf chickens together with 62 turkey sera, all of them taken from one - twelve week-old non-infected birds from different experimental studies, served as negative controls. Absence of histomonads in these birds was constantly proven by continuous cloacal swabbing and subsequent recultivations.

### Chicken sera from infected birds

Chicken sera were taken from a recently described animal trial (Hess et al., 2006a). Briefly, spf chickens (VALO, Lohmann, Cuxhaven, Germany) were infected via the cloaca at 14 days of life using a virulent isolate of H. meleagrids (Hm/Turkey/Austria/2922-66/C04). Birds were bled prior to infection and in weekly intervals up to 6 weeks post infection when the study was terminated. None of the 10 infected and the 4 in-contact birds displayed any adverse clinical signs throughout the whole experiment.

### Turkey sera from infected birds

Serum samples from a total of 52 turkeys ("B.U.T. 9") were used from a previously described infection experiment, demonstrating the benefit of vaccination (Hess et al., 2008). All birds were bled prior to vaccination performed via the cloacal route at 14 days of life with attenuated histomonads, achieved by continuous in vitro passaging of a virulent isolate. Investigated sera originated from 14 birds/group of which 10 birds were initially vaccinated either with passage (P) 95 (group I), P215 (group II) or P295 (group III) and 4 birds/group were kept as in-contact animals. Four weeks after vaccination 10 birds/group were challenged cloacally and 4 previously vaccinated birds were kept as in-contact birds. Blood samples from all of the birds were collected weekly until 4 weeks post challenge. At the same time blood samples were also taken from 10 non-vaccinated but challenged birds which were housed as a separate group IV. With the exception of one bird all birds in this group died or had to be euthanized up to two weeks post challenge allowing only two sampling dates.

Following sample collection blood samples were kept overnight at 4°. After 24h the samples were centrifuged at 3300 x g for 12 minutes (Hettich Rotanta 460), aliquoted and the sera were stored at -20° for further use.

### Preparation of the antigen for ELISA

The clonal culture, which was also used in the animal experiments, was passaged in vitro up to 25 times. The parasites were cultivated in 50 ml sterile FALCON tubes in 9 ml medium 199 HEPES (Gibco™, Invitrogen) containing 15 % foetal calf serum (Gibco™, Invitrogen) and 22 mg autoclaved rice starch (Sigma-Aldrich). The cultures were passaged every 48 or 72 hours by transferring 1 ml of the culture into 9 ml of fresh medium. For preparation of the antigen, the cultures were purified through several centrifugation steps. First the tubes were placed on a tumbler to dissolve the cell-rice pellet at the bottom of the tube. Then the culture was centrifuged at 1000 g for 5 min. Afterwards the supernatant was carefully taken off using a sterile transfer pipette and 5 ml of fresh medium was added. The cell pellet was resuspended again using the tumbler and centrifuged at the same velocity as before. These steps were repeated until the culture was washed four times with fresh medium 199. After the last centrifugation step the pellet was dissolved in 1ml medium 199, transferred to a 2 ml Eppendorf tube and centrifuged at 200 g for 2 minutes. After that the supernatant was taken off carefully and the remaining pellet resuspended in phosphate-buffered-saline pH 7.4 plus 0.05 % Tween 20 (PBST). The cells were counted in a Neubauer counting chamber and diluted in PBST to reach a final concentration of 10 000 histomonads in 100 µl. The antigen suspensions were aliquoted in 10 ml portions and stored at -20° until further use.

### ELISA procedure

The optimal dilutions for the reagents were predetermined through checker-board titrations. For the indirect sandwich ELISA 96-well ELISA plates (Nunc Medisorb®) were coated with 100 µl rabbit anti-histomonas serum in a 1:10000 dilution in carbonate buffer pH 9.6. The coated plates were left overnight and incubated at 4°. The next day the plates were washed with washing buffer (PBS containing 0.05% Tween 20, pH 7.4; 3 times 300µl/well) using an automating plate washer (IDEXX Columbus D) for removal of all unbound particles,. The coated plates were closed with a lid, sealed with laboratory Parafilm® "M" (Pechiney Plastic Packaging, Chicago, USA) and stored at -20° until further use. Plates were thawed at room temperature and incubated with 300 µl/well of blocking buffer (Starting Block™ T20 PBS, Pierce) to prevent non-specific binding. After two minutes of incubation the buffer was removed through inversion of the plates. The plates were tapped vigorously on absorptive paper to remove the remaining fluid. Afterwards 100 µl of the histomonas antigen diluted in PBST was added to each well and incubated for 1 hour at room temperature. Following antigen washing with PBST, 100 µl of the serum samples diluted 1:500 in blocking buffer were added. The plates were incubated again for 1 hour at room temperature and then washed as mentioned above. Goat-Anti-Turkey IgG-horseradish-peroxidase (HRP) or Goat-Anti-Chicken-IgG-HRP (Southern Biotechnology, USA) was added in a 1:5 000 dilution in PBST and again incubated for 1 hour. Washing was once repeated and afterwards tetramethylbenzidine (TMB) substrate (Calbiochem®) was added (100 µl/ well) and incubated for 15 minutes in the dark. The reaction was stopped with 100 µl 0.5 M sulphuric acid /well. The optical density (OD) of all wells was measured in a spectrophotometer (ELx800, Bio-TEK Instruments, INC.) at a wavelength of 450 nanometer (nm). The same procedure was applied for the indirect ELISA, except that the rabbit antiserum was omitted and the same amount of histomonas cells diluted in carbonate buffer was directly coated on the plates. Chicken and turkey sera were tested on the same set of microtiter plates coated with the rabbit serum on the same day. After coating the plates they were washed and stored at -20°. The used antigen derived of the same passage and it was purified and diluted on the same day, meaning that all tests were done with the same set of plates and antigen. Four wells/ plate were used to incorporate always the same two positive and negative control sera in all assays in order to prove the overall test performance. All sera were tested in duplicate.

### Coefficient of variation

To determine the repeatability of the test, five preparations of antigen samples obtained from the same passage (P25), were processed on different occasions and tested accordingly. As primary antibody a set of 20 randomly chosen sera obtained from non-infected chickens and turkeys were tested on each of the 5 plates. The mean of every sample set was determined and the standard deviation of the mean values of the 5 replicates was calculated and put in relation to the overall mean of the replicates. To ensure an adequate repeatability, the allegation was that coefficients of variation should be equal or less than 10 % (Crowther, 2000).

### Determination of the cut-off value

The arithmetic means of all OD values plus 3 times the standard deviations determined separately from all non-infected chicken or turkey sera were used to establish the cut-off values. In chickens OD values above 0.54 were considered positive, whereas in turkeys a cut-off value of 0.36 was determined. To demonstrate the benefit of the indirect sandwich ELISA system the same testing was performed in parallel with the indirect ELISA, where the plates were coated directly with the same amount of antigen without the rabbit serum, and the mean values and standard deviations of the results of both tests were compared. All statistics were performed with SPSS 14.0 (SPSS Inc., Chicago, USA).

### Example 2: Test performance of the ELISA

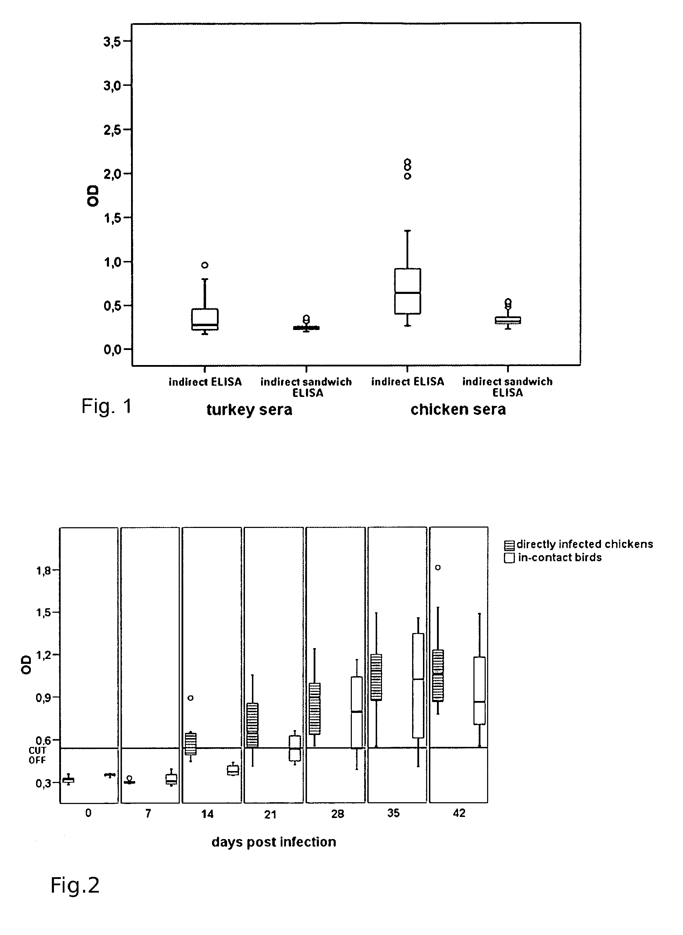

The first comparative experiments were performed with an indirect ELISA by coating the wells directly with the histomonas antigen. The polyclonal rabbit serum raised against *H. meleagridis* was used as primary antibody in order to control the coating procedure, as high OD values were achieved. However, processing turkey and chicken sera from non-infected birds via the indirect ELISA, these control sera showed a high background signal. To minimize these background signals the plates were first of all coated with the polyclonal anti-histomonas rabbit serum, moving towards an indirect sandwich system. Following this approach the background signals were significantly reduced as displayed by a low mean value and a lower standard deviation obtained from the negative sera (Fig. 1).

Comparing 5 different antigen preparations and testing of 20 negative sera OD values ranged from 0,298- 0,344 and 0,304-0,317 for the chicken and turkey sera, respectively. Thus a coefficient of variation of 5.32 % and 1.73 % was obtained.

### Example 3: Analysis of the chicken and turkey sera from experimentally infected birds by the indirect sandwich ELISA

### Chicken sera

Testing of sera started prior to the infection (day 0). An antibody titer above the determined cut-off value was first noticed on the 14th day post infection (d.p.i.) in some of the directly infected animals whereas antibodies in the in-contact birds stayed below the cut-off value (Fig. 2). A rise of antibodies was noticed at 21 d.p.i. and all directly infected birds were above the cut-off value at 28 d.p.i. whereas the in-contact birds showed first positive titers at 21st day p.i.. However, until the end of the experiment all in-contact birds displayed a clear positive extinction.

### Turkey sera

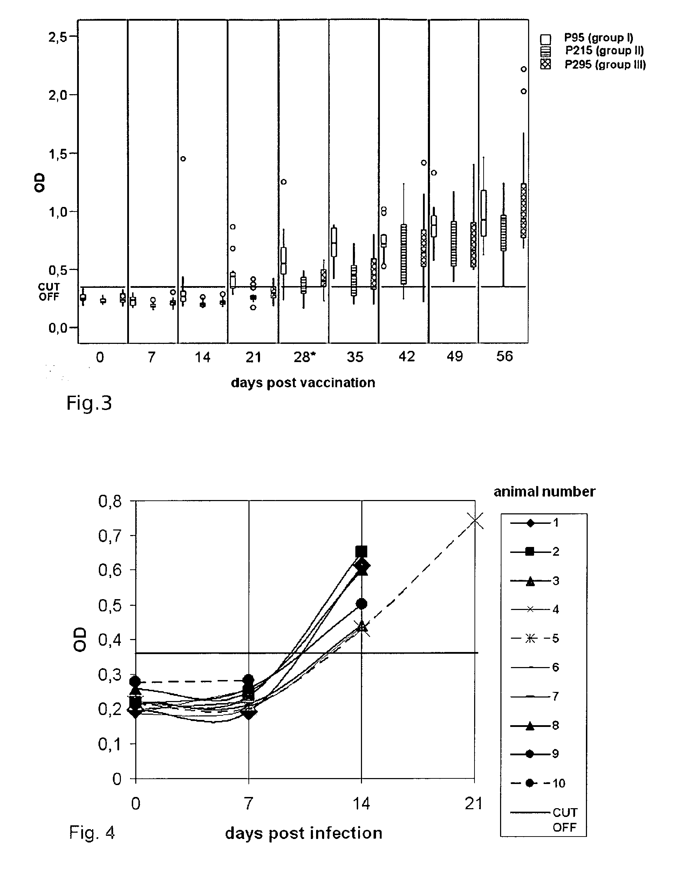

First measurement started prior to vaccination of birds at 14 days of life (day 0). Within the next two weeks all birds vaccinated with one of the higher passages (P215 or P295) did not develop an antibody response above the cut-off value. In comparison, birds from group I vaccinated with the lowest passage (P95) reacted earlier to the antigen, with the first noticeable OD values above the cut-off on the 14th day post vaccination (p.v.). All birds in group I developed a positive antibody reaction 1 week post challenge and the OD values increased only slightly until the termination of the experiment. In group II vaccinated with P215 first OD values above the cut-off were noticed 3 weeks p.v.. Furthermore, it took up to day 49 p.v. which is three weeks post challenge (p.c.) until all birds were tested positive, displaying a somewhat delayed reaction in comparison with group I. Birds from group III infected with the highest passage number (P295) showed a similar reaction pattern as described for the birds in group II and the first positive samples were noticed on day 21 p.v.. At the end of the experiment and 4 weeks p.c. the turkeys of this group displayed the highest extinctions (Fig. 3).

An antibody response above the cut-off value was also noticed in all non-vaccinated birds of group IV which survived up to 14 d.p.i. (Fig. 4). No further measurement was possible as all birds died by histomonosis during the next few days. Exceptional to this, one bird (no. 5) survived until day 23 displaying a further rise of antibodies.

### Example 4: Discussion and conclusion

No serological method suitable for large screening of antibodies against *Histomonas meleagridis* in poultry is available so far. According to the invention, an immunoassay, in particular an ELISA, was developed as a suitable tool for this purpose. Investigating serum samples from non-infected specific pathogen-free (spf) chickens and commercial turkeys a high background signal was noticed when ELISA plates were directly coated with purified parasitic cells. This signal was significantly reduced by coating the plates with a polyclonal rabbit antibody, raised against histomonads, prior to the addition of the antigen. Adopting this approach five antigen preparations were compared and a high reproducibility could be demonstrated reflected by a very low coefficient of variation of 5,3% and 1,7% for the chicken and turkey sera, respectively. After this initial development all further experiments were carried out with one set of plates and the same antigen preparation. Investigating chicken sera obtained from birds infected at 14 days of life, OD values above a predetermined cut-off value were observed two weeks post infection and a rise of IgG antibodies was noticed until 6 weeks post infection, when the experiment was terminated. Non-protected turkeys infected at the same age displayed an increasing IgG response until 14 days of life, prior to the death of animals due to histomonosis. In comparison, the majority of turkeys vaccinated with attenuated histomonads, obtained through prolonged passaging and challenged four weeks later with virulent parasites, displayed a demonstrable antibody response after the challenge only. Antibody titers increased until 4 weeks post challenge when the birds were killed and the study was terminated. Altogether, the developed indirect sandwich ELISA proved to be a quick and efficient method to detect IgG antibodies against *H. meleagridis* in sera of experimentally infected chickens and turkeys and will be a helpful tool to obtain more insights into the epidemiology of the parasite and the immune response of its hosts.

Serum samples were tested, obtained from experimentally infected spf chickens and commercial turkeys.
Investigating the chicken sera antibody titers above the cut-off value were noticed 14 d.p.i. In the present study higher mean titers were measured in the birds vaccinated with the least attenuated protozoa. Such histomonads have obviously kept the ability to penetrate the intestinal mucosal barrier, an obvious distinction between virulent and attenuated isolates. The fact that all non-vaccinated birds which died after the second blood sampling at 14 days post challenge expressed an antibody titer above the cut-off value is also an indicator for the relationship between virulence and antibody response.

The increase of antibodies following challenge was most severe in the two groups vaccinated with the more attenuated histomonads. Interestingly, in these groups some birds were noticed which did not display an antibody response above the cut-off level prior to challenge. Anyhow, following challenge none of these birds suffered or died from histomonosis indicating that humoral antibodies are of limited value for protection. This is also in agreement with earlier tests, which failed to protect turkeys by transferring serum from immune to susceptible birds.

Although IgG antibodies may not play a major role in the development of a protective immunity against the protozoan parasite, they present a good option to determine the infectious status of poultry, especially chickens as the development of clinical signs and post mortem lesions is much less severe in these birds. Based on the presented data clinical asymptomatic, infected animals can be identified starting 14 days post infection.

### References

Crowther, Methods Mol.Biol., 149, III-413 (2000) Hess et al., Avian Pathol., 35, 280-285 (2006a) Hess et al., Parasitology, 133, 547-554 (2006b) Hess et al., Vaccine, 26, 4187-4193 (2008) Singh et al., Exp.Parasitol., 118, 505-513 (2008)

## Claims

1. A method for the detection or quantification of antibodies against *Histomonas meleagridis* in a sample comprising
• providing a carrier surface (a) with immobilized antibodies against *Histomonas meleagridis* and *Histomonas meleagridis* antigens immobilized on said antibodies, and/or (b) with isolated *Histomonas meleagridis* antigens,
• contacting a sample which potentially comprises antibodies against *Histomonas meleagridis,* and
• detecting binding events of the antibodies of the sample onto the carrier surface.

2. Method according to claim 1, **characterized in that** the *Histomonas meleagridis* antigens immobilized via the antibody (a) are *Histomonas meleagridis* cells or cell lysate antigens.

3. Method according to claim 1 or 2, **characterized in that** the immobilized antibodies (a) are of a different organism than the sample, preferably the immobilized antibodies (a) are of a mammal, preferably a rabbit.

4. Method according to any one of claims 1 to 3, **characterized in that** the binding event is detected by competitive binding of a labeled antibody, preferably fluorescence labeled or enzyme labeled antibody, being specific for immobilized antigens (a) or (b), to the antigens (a) or (b) of the carrier surface during or after the contacting step with the sample.

5. Method according to any one of claims 1 to 4, **characterized in that** the binding event is detected by secondary antibodies which are specific for the Fc portion of the antibodies to be detected in the sample, and said secondary antibodies are labeled, preferably fluorescence labeled or enzyme labeled.

6. Method according to any one of claims 1 to 5, **characterized in that** the sample is a blood, blood plasma or blood serum sample.

7. Method according to any one of claims 1 to 6, **characterized in that** the *Histomonas meleagridis* antigens immobilized on said antibodies (a) or isolated *Histomonas meleagridis* surface antigens (b) are of a clonal culture of *Histomonas meleagridis,* preferably the culture of *Histomonas meleagridis* is of Hm/Turkey/Austria/2922-C6/04, Hm/Turkey/Austria/5642-C4/05, Hm/Turkey/Austria/2877-C3/05, Hm/Turkey/Austria/2877-C4/05, Hm/Turkey/Austria/2877-C7/05, Hm/Turkey/Austria/2877-C11/05, Hm/Turkey/Austria/2877-C17/05, Hm/Turkey/Germany/4114-C3/05, Hm/Turkey/Germany/4114-C16/05, Hm/Turkey/Germany/4114-C18/05, Hm/Chicken/Hungary/5009-C2/05, Hm/Chicken/Hungary/5009-C7/05, Hm/Chicken/Hungary/5009-C10/05, Hm/Chicken/Hungary/5009-C13/05, Hm/Chicken/Hungary/5009-C14/05, Hm/Chicken/Austria/8175-C2/06, Hm/Chicken/Austria/8175-C3/06, Hm/Chicken/Austria/8175-C5/06, Hm/Chicken/Austria/8175-C7/06, Hm/Chicken/Austria/8175-C18/06, Hm/Chicken/Austria/8175-C20/06, Hm/Turkey/Austria/2877-CH3/05, Hm/Turkey/Austria/2877-C10/05.

8. A method for the diagnosis of an *Histomonas meleagridis* infection in an animal, preferably a bird, in particular preferred chicken, turkey, peafowl, quail, duck or pheasant, comprising
• providing a blood sample of the animal,
• detecting or quantifying antibodies against *Histomonas meleagridis* in said sample according to a method of any previous claim, and
• comparing amounts of the detected of quantified antibody with antibody amounts of a confirmed infectious state or confirmed non-infectious state amount, thereby diagnosing the *Histomonas meleagridis* infection in the animal.

9. A carrier surface with (a) immobilized antibodies against *Histomonas meleagridis* and *Histomonas meleagridis* antigens immobilized on said antibodies, and/or (b) isolated *Histomonas meleagridis* surface antigens.

10. Carrier surface according to claim 9 being a solid surface or a semi-solid surface, preferably a gel.

11. Carrier surface according to claim 9 or 10, **characterized in that** the *Histomonas meleagridis* antigens immobilized via the antibody (a) are *Histomonas meleagridis* cells or cell lysate antigens.

12. Carrier surface according to claim 9 or 10, **characterized in that** the isolated surface antigens (b) are purified or recombinant antigens.

13. A kit suitable for a method according to claim 1 comprising a carrier surface according to any one of claims 9 to 12 and detection means for the detection of binding events of antibodies onto the immobilized antigens (a) or (b) of the carrier surface.

14. Kit according to claim 13 wherein said detection means are labeled competitive antibodies, preferably fluorescence labeled or enzyme labeled antibodies, being specific for immobilized antigens (a) or (b) or labeled secondary antibodies, preferably fluorescence labeled or enzyme labeled antibodies, being specific for the Fc potion of antibodies of an organism expected to be in a sample for which the kit is intended, preferably secondary labeled anti-chicken or anti-turkey antibodies, optionally further comprising sample holding means, preferably sample isolation and holding means, in particular a syringe.

15. Use of a carrier surface according to any one of claims 9 to 12 or a kit according to any one of claims 13 to 14 in a method according to any one of claims 1 to 8.
